(19) Europäisches Patentamt European Patent Office Office européen des brevets

(11) **EP 4 718 291 A1**

(12) **EUROPEAN PATENT APPLICATION**

(43) Date of publication:
**01.04.2026 Bulletin 2026/14**

(21) Application number: **25202781.8**

(22) Date of filing: **17.09.2025**

(51) International Patent Classification (IPC):
**G06F 18/28** (2023.01)    **G06V 10/82** (2022.01)
**G06V 20/69** (2022.01)

(52) Cooperative Patent Classification (CPC):
**G06V 10/82; G06F 18/28; G06V 20/69**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **27.09.2024 IN 202421073366**

(71) Applicant: **Tata Consultancy Services Limited Maharashtra (IN)**

(72) Inventors:
• **REDDY KANCHAM, Pavan Kumar**
  **560066 Bangalore, Karnataka (IN)**
• **GUBBI LAKSHMINARASIMHA, Jayavardhana Rama**
  **560066 Bangalore, Karnataka (IN)**
• **MAKADIYA, Shwet Denish**
  **560066 Bangalore, Karnataka (IN)**

(74) Representative: **Goddar, Heinz J.**
**Boehmert & Boehmert**
**Anwaltspartnerschaft mbB**
**Pettenkoferstrasse 22**
**80336 München (DE)**

(54) **EFFICIENT AGGREGATION METHOD AND SYSTEM FOR MULTIPLE INSTANCE LEARNING**

(57)    Multiple Instance Learning (MIL) is a useful method for extracting information from Gigapixel images using weakly supervised learning. Traditionally, it was approached with the hypothesis of considering each instance as an independent and identically distributed entity. Recently, it has been suggested that correlations between different instances would yield better results for classification. The present disclosure is designed to improve classification performance significantly. The self-selective MIL of the present disclosure facilitates binary classification with enhanced insights into more aggressive tumor regions.

FIG. 1B

## Description

CROSS-REFERENCE TO RELATED APPLICATIONS AND PRIORITY

The present application claims priority from Indian patent application no. 202421073366, filed on September 27, 2024.

TECHNICAL FIELD

The disclosure herein generally relates to the field of Machine Learning (ML) and, more particularly, to an efficient aggregation method and system for multiple instance learning.

BACKGROUND

Whole Slide Images (WSI) offer a valuable means of capturing tissue information for examination at the cellular level. However, it is worth noting that WSI images typically come in gigapixel sizes. When considering histopathology WSI classification, there are two primary types: (i) distinguishing between normal tissue and tumors and (ii) distinguishing between different types of tumors. Different subtypes of tumors require different therapeutic approaches. Therefore, for improved prognosis, accurate classification of tumor sub-types is crucial.

SUMMARY

Embodiments of the present disclosure present technological improvements as solutions to one or more of the above-mentioned technical problems recognized by the inventors in conventional systems. For example, in one embodiment, an efficient aggregation method for multiple instance learning is provided. The method includes receiving, via one or more hardware processors, a plurality of Whole Slide Images (WSIs) corresponding to a plurality of subjects. Further, the method includes generating, by the one or more hardware processors, a plurality of non-overlapping patch instances from each of the plurality of WSIs, wherein a background associated with each of the plurality of non-overlapping patch instances were removed. Furthermore, the method includes extracting, by the one or more hardware processors, a plurality of features from the plurality of patch instances associated with each of the plurality of WSI, using a Deep Neural Network (DNN). Furthermore, the method includes computing, by the one or more hardware processors, a plurality of feature embeddings based on the extracted plurality of features using a Machine Learning (ML) model. Finally, the method includes training, by the one or more hardware processors, a transformed-based Multiple Instance Learning (MIL) model based on the plurality of feature embeddings associated with each of the plurality of patch instances by: (i) generating a first triple vector comprising a Query (Q), a Value (V), and a Key (K) by performing linear projection on the plurality of feature embeddings (ii) computing a first plurality of correlation scores between each feature embedding from among the plurality of feature embeddings with other plurality of feature embeddings from among the plurality of feature embeddings based on the first triple vector (iii) selecting a first optimal feature embedding from among the plurality of feature embeddings based on the first plurality of correlation scores, wherein the feature embedding with maximum correlation score is selected as the optimal feature embedding (iv) generating a second triple vector comprising the Query (Q), the Value (V), and a Key (K) by performing linear projection on the plurality of feature embeddings and the first optimal feature embedding (v) computing a second plurality of correlation scores between each feature embedding from among the plurality of feature embeddings and the first optimal feature embedding based on the second triple vector (vi) selecting a second optimal feature embedding from among the plurality of feature embeddings based on the second plurality of correlation scores, wherein the feature embedding with maximum correlation score is selected as the second optimal feature embedding (vii) generating a third triple vector comprising the Query (Q), the Value (V), and a Key (K) by performing linear projection on the plurality of feature embeddings and the second optimal feature embedding (viii) computing a third plurality of correlation scores between each feature embedding from among the plurality of feature embeddings and the second optimal feature embedding based on the third triple vector; and (ix) selecting a third optimal feature embedding from among the plurality of feature embeddings based on the third plurality of correlation scores, wherein the feature embedding with maximum correlation score is selected as the third optimal feature embedding.

In another aspect, an efficient aggregation system for multiple instance learning is provided. The system includes at least one memory storing programmed instructions, one or more Input /Output (I/O) interfaces, and one or more hardware processors operatively coupled to the at least one memory, wherein the one or more hardware processors are configured by the programmed instructions to receive a plurality of Whole Slide Images (WSIs) corresponding to a plurality of subjects. Further, the one or more hardware processors are configured by the programmed instructions to generate a plurality of non-overlapping patch instances from each of the plurality of WSIs, wherein a background associated with each of the plurality of non-overlapping patch instances were removed. Furthermore, the one or more hardware processors are configured by the programmed instructions to extract a plurality of features from the plurality of patch instances associated with each of the plurality of WSI, using a Deep Neural Network (DNN). Furthermore, the one or more hardware processors are configured by the programmed instructions to compute a plurality of feature embeddings based on the extracted plurality of features using a Machine Learning (ML) model. Finally, the one or more hardware processors are configured by the programmed instructions to train a transformed-based Multiple Instance Learning (MIL) model based on the plurality of feature embeddings associated with each of the plurality of patch instances by: (i) generating a first triple vector comprising a Query (Q), a Value (V), and a Key (K) by performing linear projection on the plurality of feature embeddings (ii) computing a first plurality of correlation scores between each feature embedding from among the plurality of feature embeddings with other plurality of feature embeddings from among the plurality of feature embeddings based on the first triple vector (iii) selecting a first optimal feature embedding from among the plurality of feature embeddings based on the first plurality of correlation scores, wherein the feature embedding with maximum correlation score is selected as the optimal feature embedding (iv) generating a second triple vector comprising the Query (Q), the Value (V), and a Key (K) by performing linear projection on the plurality of feature embeddings and the first optimal feature embedding (v) computing a second plurality of correlation scores between each feature embedding from among the plurality of feature embeddings and the first optimal feature embedding based on the second triple vector (vi) selecting a second optimal feature embedding from among the plurality of feature embeddings based on the second plurality of correlation scores, wherein the feature embedding with maximum correlation score is selected as the second optimal feature embedding (vii) generating a third triple vector comprising the Query (Q), the Value (V), and a Key (K) by performing linear projection on the plurality of feature embeddings and the second optimal feature embedding (viii) computing a third plurality of correlation scores between each feature embedding from among the plurality of feature embeddings and the second optimal feature embedding based on the third triple vector; and (ix) selecting a third optimal feature embedding from among the plurality of feature embeddings based on the third plurality of correlation scores, wherein the feature embedding with maximum correlation score is selected as the third optimal feature embedding.

In yet another aspect, there are provided one or more non-transitory machine-readable information storage mediums comprising one or more instructions which when executed by one or more hardware processors cause: receiving a plurality of Whole Slide Images (WSIs) corresponding to a plurality of subjects. Further, the one or more non-transitory machine-readable information storage mediums comprising one or more instructions which when executed by one or more hardware processors cause generating a plurality of non-overlapping patch instances from each of the plurality of WSIs, wherein a background associated with each of the plurality of non-overlapping patch instances were removed. Furthermore, the one or more non-transitory machine-readable information storage mediums comprising one or more instructions which when executed by one or more hardware processors cause extracting a plurality of features from the plurality of patch instances associated with each of the plurality of WSI, using a Deep Neural Network (DNN). Furthermore, the one or more non-transitory machine-readable information storage mediums comprising one or more instructions which when executed by one or more hardware processors cause computing a plurality of feature embeddings based on the extracted plurality of features using a Machine Learning (ML) model. Finally, the one or more non-transitory machine-readable information storage mediums comprising one or more instructions which when executed by one or more hardware processors cause training a transformed-based Multiple Instance Learning (MIL) model based on the plurality of feature embeddings associated with each of the plurality of patch instances by: (i) generating a first triple vector comprising a Query (Q), a Value (V), and a Key (K) by performing linear projection on the plurality of feature embeddings (ii) computing a first plurality of correlation scores between each feature embedding from among the plurality of feature embeddings with other plurality of feature embeddings from among the plurality of feature embeddings based on the first triple vector (iii) selecting a first optimal feature embedding from among the plurality of feature embeddings based on the first plurality of correlation scores, wherein the feature embedding with maximum correlation score is selected as the optimal feature embedding (iv) generating a second triple vector comprising the Query (Q), the Value (V), and a Key (K) by performing linear projection on the plurality of feature embeddings and the first optimal feature embedding (v) computing a second plurality of correlation scores between each feature embedding from among the plurality of feature embeddings and the first optimal feature embedding based on the second triple vector (vi) selecting a second optimal feature embedding from among the plurality of feature embeddings based on the second plurality of correlation scores, wherein the feature embedding with maximum correlation score is selected as the second optimal feature embedding (vii) generating a third triple vector comprising the Query (Q), the Value (V), and a Key (K) by performing linear projection on the plurality of feature embeddings and the second optimal feature embedding (viii) computing a third plurality of correlation scores between each feature embedding from among the plurality of feature embeddings and the second optimal feature embedding based on the third triple vector; and (ix) selecting a third optimal feature embedding from among the plurality of feature embeddings based on the third plurality of correlation scores, wherein the feature embedding with maximum correlation score is selected as the third optimal feature embedding.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1A is a functional block diagram of an efficient aggregation system for multiple instance learning, in accordance with some embodiments of the present disclosure.
FIGS. 1B through 1F illustrates overall functional architecture of the efficient aggregation system for the multiple instance learning, in accordance with some embodiments of the present disclosure.
FIGS. 2A, 2B and 2C illustrates a flow diagram for a processor implemented efficient aggregation method for multiple instance learning, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

The absence of pixel-level or patch-level labeling poses a challenge, which makes it difficult to apply deep learning techniques in Whole Slide Images (WSI). This challenge arises because using the WSI image directly would demand substantial computational power, while down-sampling the WSI introduces a significant risk of information loss during the process. This is where Multiple Instance Learning (MIL) steps play a crucial role.

Initially, MIL was proposed as an alternative to supervised learning, specifically for scenarios involving incomplete knowledge of training sample labels. When using MIL with WSI, it takes a WSI and partitions it into distinct patches, referred to as instances, and all the instances from a specific WSI collectively form a bag of instances. However, in supervised learning of WSI, labeling each of these patches is challenging and time-consuming as the number of instances in a bag can be in the order of thousands. Therefore, the present disclosure chosen unsupervised learning for WSI, where the label is available for the entire WSI but not for each individual instance.

Currently, in instance-based MIL, the WSI is converted into multiple patches, each treated as an individual instance. Subsequently, a Deep Neural Network (DNN) is trained at the instance level, where each instance is assigned, a label corresponding to the bag label. From this pool of instances, a subset of the top representatives is selected, and aggregation is performed using these instances. However, instance-based methods typically necessitate a larger number of training WSIs , as the selection of top instances from each bag results in reduced training data.

Subsequently, embedding-based MIL was introduced, which involves converting the whole slide images into patches and then transforming these patches to fixed-dimensional embedding. Various aggregation operations, such as max-pooling or attention mechanisms, are applied to these embedding to predict the bag label. Initially, Pooling based MIL models applied max pooling to each instance-level feature embedding extracted from DNNs. Further, Attention-based MIL (AB-MIL) has proven beneficial in enhancing performance by using an aggregation operator parameterized by neural networks, which incorporates the contribution of each instance to the bag embedding. Thereafter, Dual-Stream MIL (DSMIL) approach pioneered the use of non-local attention blocks, which aim to select the top instance using instance-based MIL and then correlate it with the remaining instances. In DS-MIL it was observed that employing max pooling could facilitate the selection of the instance with the highest probability related to the tumor. Moreover, by calculating the similarity between the highest scored instance and the remaining instances, improved performance could be achieved. This finding somewhat contradicted the initial hypothesis that MIL problems should be treated as instances of independent and identical distribution.

Furthermore, Correlation based MIL leveraged the correlation between different instances as a method for morphological learning. Each instance is regarded as correlated not only with its surroundings but also with instances even from distant positions. In TransMI, it has been demonstrated that for WSI image classification, it is crucial to take into account contextual information within a specific area, as well as correlation information between different areas. Therefore, employing self-attention mechanisms can enhance the understanding of both local and long-range correlation information.

Finally, Two-tier based methods adopt a different approach by first eliminating least important instances using MIL, considering only the most prominent representatives of all instances and applying MIL on those selected instances. DTFD-MIL introduced the concept of pseudo-bags, where all the instances of a particular WSI are divided into multiple sub-groups called pseudo bags and aimed to select the best features from all instances within a pseudo-bag using AB-MIL. These selected features were then utilized for attention-based aggregation, resulting in improved results. Consequently, this method overlooks the correlations between some instances in a pseudo-bag with those in other pseudo bags, which might lead to information loss, especially if they are not selected as the top representatives within their respective pseudo-bags.

When considering the correlation among all instances, it is important to acknowledge the presence of positive instances, uncertain instances and negative instances. Out of which, uncertain instances can potentially degrade correlation scores and introduce information complexity. Thus, for excluding these uncertain instances and focusing solely on certain information, it is necessary to enhance correlation scores and consequently improve classification accuracy.

To overcome this drawback of the conventional approaches, the present disclosure selects those positive instances and correlate the feature of those selected positive instance with other instances. As a result, with the present disclosure, it was observed that the differentiation of the negative instances from the selected positive instances helps in classifying those uncertain instances. Classification of uncertain instances thus helps in classifying the remaining into either of the classes and lower down the importance of negative instances. The present disclosure focuses on enhancing the sub-classification of tumors rather than distinguishing between normal tissue and cancerous tumor tissue.

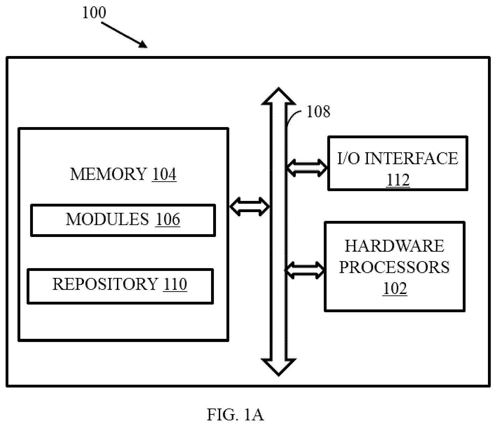

Referring now to the drawings, more particularly to FIG. 1A through FIG. 2C, where similar reference characters denote corresponding features consistently throughout the figures, there are shown preferred embodiments, and these embodiments are described in the context of the following exemplary system and/or method.

FIG. 1A is a functional block diagram of an efficient aggregation system 100 for multiple instance learning, in accordance with some embodiments of the present disclosure. The system 100 includes or is otherwise in communication with hardware processors 102, at least one memory such as a memory 104, an Input /Output (I/O) interface 112. The hardware processors 102, memory 104, and the I/O interface 112 may be coupled by a system bus such as a system bus 108 or a similar mechanism. In an embodiment, the hardware processors 102 can be one or more hardware processors.

The I/O interface 112 may include a variety of software and hardware interfaces, for example, a web interface, a graphical user interface, and the like. The I/O interface 112 may include a variety of software and hardware interfaces, for example, interfaces for peripheral device(s), such as a keyboard, a mouse, an external memory, a printer and the like. Further, the I/O interface 112 may enable system 100 to communicate with other devices, such as web servers, and external databases.

The I/O interface 112 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 112 may include one or more ports for connecting several computing systems with one another or to another server computer. The I/O interface 112 may include one or more ports for connecting several devices to one another or to another server.

The one or more hardware processors 102 may be implemented as one or more microprocessors, microcomputers, microcontrollers, digital signal processors, central processing units, node machines, logic circuitries, and/or any devices that manipulate signals based on operational instructions. Among other capabilities, the one or more hardware processors 102 is configured to fetch and execute computer-readable instructions stored in memory 104.

The memory 104 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, memory 104 includes a plurality of modules 106. Memory 104 also includes a data repository (or repository) 110 for storing data processed, received, and generated by the plurality of modules 106.

The plurality of modules 106 includes programs or coded instructions that supplement applications or functions performed by the efficient aggregation system 100 for Multiple instance learning. The plurality of modules 106, amongst other things, can include routines, programs, objects, components, and data structures, which perform particular tasks or implement particular abstract data types. The plurality of modules 106 may also be used as, signal processor(s), node machine(s), logic circuitries, and/or any other device or component that manipulates signals based on operational instructions. Further, the plurality of modules 106 can be used by hardware, by computer-readable instructions executed by the one or more hardware processors 102, or by a combination thereof. The plurality of modules 106 can include various sub-modules (not shown). The plurality of modules 106 may include computer-readable instructions that supplement applications or functions performed by the efficient aggregation system 100 for Multiple instance learning.

The data repository (or repository) 110 may include a plurality of abstracted pieces of code for refinement and data that is processed, received, or generated as a result of the execution of the plurality of modules in the module(s) 106.

Although the data repository 110 is shown internal to the system 100, it will be noted that, in alternate embodiments, the data repository 110 can also be implemented external to the system 100, where the data repository 110 may be stored within a database (repository 110) communicatively coupled to the system 100. The data contained within such an external database may be periodically updated. For example, new data may be added into the database (not shown in FIG. 1A) and/or existing data may be modified and/or non-useful data may be deleted from the database. In one example, the data may be stored in an external system, such as a Lightweight Directory Access Protocol (LDAP) directory, or a Relational Database Management System (RDBMS).

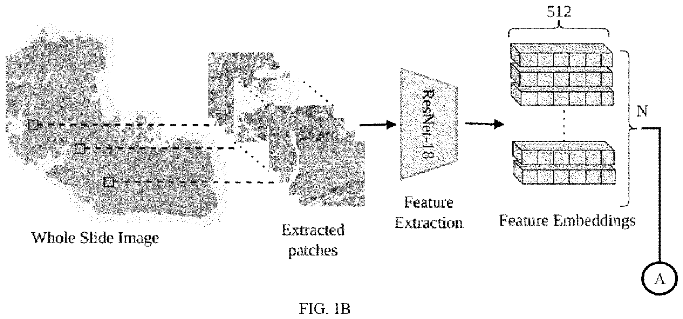

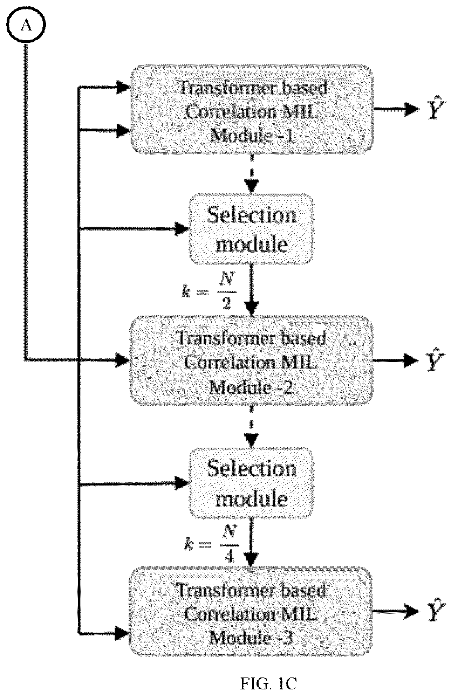

The overall architecture of the system of FIG. 1A is explained in conjunction with FIGS. 1B through 1F. Now referring to figures, FIG. 1B and FIG. 1C collectively illustrate the overall functional architecture of the present disclosure. Now referring to FIG.1B and FIG. 1C, the process begins by inputting the sequence into the first correlation module, where the weights for each instance are stored as attention scores. These scores determine the relevance of each instance in predicting the bag label. Further, these attention scores are arranged in descending order and the top N/2 instances are chosen with the highest scores to predict the bag label.

When considering binary MIL classification, the aim is to predict a target value $Y_i = \{0,1\}$ from given bag of instances $X_i = \{x_{i,1}, x_{i,2}, ... ... ... ... x_{i,n}\}$, where n represents the number of instances in the $i^{th}$ bag, each of which exhibits dependency on one another within the bag. Here, $i$ ranges from 1 to $I$, where $I$ denotes the number of WSIs or bags. In this scenario, the instance-level labels are unknown, denoted as $Y_i = \{y_{i,1}, y_{i,2}, \cdots \cdots ... ... y_{i,n}\}$,, with only the bag-level label $Y_i$ being provided for each bag. Binary MIL can thus be defined as given in (1).

$$Y_i = \begin{cases} 0, & if \ \sum y_i = 0 \\ 1, & otherwise \end{cases} \quad \dots\dots\dots\dots\dots\dots\dots\dots\dots\dots\dots(1)$$

When considering correlated MIL in classification, the aim is to predict class value $Y_i = \{0,1\}$ from given bag of instances $X_i = \{x_{i,1}, x_{i,2}, ... ... ... ... x_{i,n}\}$, as given in Pseudocode 1. Now referring to pseudocode 1, the initial step involves extracting morphological learning. This is accomplished by employing the function, denoted as $f$, which utilizes multi-head self-attention mechanisms. It correlates the features extracted from each patch with those of all the other patches by employing queries and keys matrix multiplication. Subsequently, the value matrix is adjusted based on these correlation scores. Finally, utilizing an aggregation function denoted as $g$, the aim is to consolidate the morphological learning into class label.

## Pseudocode 1: A generic three-step approach for correlated MIL

**Input:** The bag of instances $X_i = \{x_{i,1}, x_{i,2}, ... ... ... ... x_{i,n}\}$

**Output:** Bag-level predicted label $\widehat{Y_i}$

1: Extract morphological information of all the instances by $f$;

2: $\quad X_f \leftarrow f(X_i)$, where $X_f \in R^{nXd}$

3: Transform $S_f$ by $g$ to obtain the predicted bag-level label;

4: $\quad \widehat{Y_i} \leftarrow g(X_f)$,

Transformer utilizes a self-attention mechanism to model the interactions among all tokens in a sequence. Therefore, it's advantageous to introduce the transformer into the correlated MIL problem. For example, given a set of bags $X_1, X_2, ..... X_b$ where each bag $X_i$ contains multiple instances $x_{i,1}, x_{i,2}, ... ... ... ... x_{i,n}$ and a corresponding label $Y_i$. The goal is to learn the mappings: $X \to T \to Y$, where $X$ is the bag space, $T$ is the Transformer space, and $Y$ is the label space.

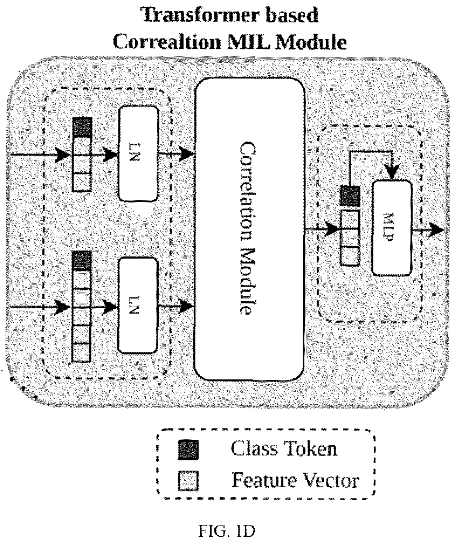

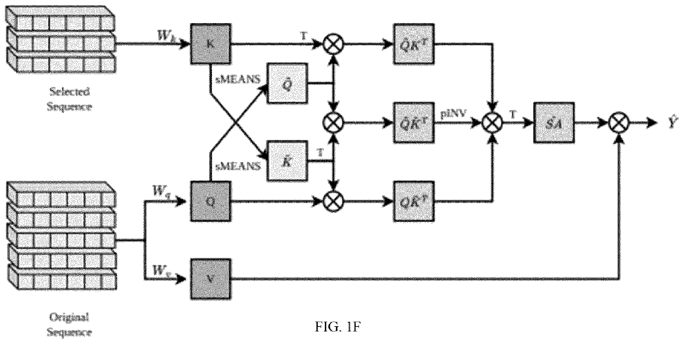

Correlation Module: In this module shown in FIG. 1D, attention similar to its application is used in many trans formers designed for Vision tasks. Here the method works with two input sequences: the original sequence and the selected sequence as shown in FIG. 1F (which would original sequence for first correlation module). The original sequence undergoes linear projection to derive Query ($Q$) and Value ($V$), while the selected sequence is linearly projected to obtain Key ($K$). The conventional attention mechanism typically involves computing similarity scores between every pair of tokens within a sequence, leading to high memory and time complexity. To tackle these challenges, especially concerning long sequences of embedding in WSI, the attention mechanism in the correlation module adopts the Nystrom Method. This method utilizes a row-by-row softmax normalization function, as stated in Equation 2.

$$\widehat{SA} = softmax\left(Q\widetilde{K}^T\middle|\sqrt{d_q}\right)\left(softmax(\tilde{Q}\widetilde{K}^T\middle|\sqrt{d_q})\right)^+ softmax\left(\tilde{Q}K^T\middle|\sqrt{d_q}\right)$$

$$\dots\dots\dots\dots\dots\dots(2)$$

Where Q~ and K~ are the m selected landmarks from the original n dimensional sequence of Q and K, and $(\cdot)+$ is the Moore-Penrose pseudoinverse. Pseudocode 2 explains a pipeline for correlation module of SoftMax matrix in self-attention.

**Pseudocode 2:**

**Input:** Original sequence of instances $X_i = \{x_{i,1}, x_{i,2}, \ldots \ldots \ldots x_{i,n}\}$ and Selected sequence of instances $S_i = \{s_{i,1}, s_{i,2}, \ldots \ldots \ldots s_{i,n}\}$

**Output:** Bag-level predicted label $\widehat{Y_i}$

1. The Original sequence of instances $(X_i)$ is projected using two matrices $W_Q \in R^{nXd}$ and $W_V \in R^{nXd}$ to get feature representations $Q$ and $V$, referred to as query, and value;

2. $Q = X_i . W_Q, \; V = X_i . W_v$

3. The Selected sequence of instances $(S_i)$ is projected using matrix $W_k \in R^{sXd_e}$, to get feature representations $K$, referred to as key;

4. $K = S_i . W_k$

5. Compute landmarks from the inputs $Q$ and $K$, to get $\tilde{Q}$ and $\widetilde{K}$ as the resultant matrix.

6. Compute $\hat{F} = softmax\left(Q\widetilde{K}^T \middle| \sqrt{d_q}\right), \hat{B} = softmax\left(\tilde{Q}K^T \middle| \sqrt{d_q}\right)$

7. Compute $\hat{A} = \left(softmax(\tilde{Q}\widetilde{K}^T \middle| \sqrt{d_q})\right)^+$

8. Compute $\widehat{SA} = \hat{F} \; X \; \hat{A} \; X \; \tilde{B}$

9. Return $\widetilde{SA} \; X \; V$

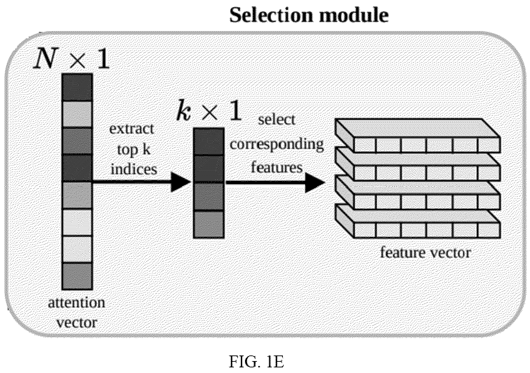

Moving forward, two input sequences were introduced to the second correlation module. One sequence contains the original input, while the other includes the selected instances (as shown in FIG 1E) from the first correlation module based on their attention scores. This process of storing attention scores and predicting the bag label using the second correlation module is repeated, however, this time the selection is refined further using the selection module shown in FIG. 1E. The selection instance is reduced to N/4 instances from the initial N/2 by picking those with the highest attention scores while predicting the bag label using the second correlation module. Continuing this pattern, two input sequences are introduced to the third correlation module: the original sequence and the selected instances from the second correlation module, based on their attention scores. Utilizing these inputs, the aim is to predict the bag label using the third correlation module. It's noteworthy that all correlation modules share the same architecture, as described in FIG. 1C. The above method is illustrated using Pseudocode 3.

## Pseudocode 3: Three-step approach for selective correlated MIL

**Input:** The bag of instances $K_i = \{k_{i,1}, k_{i,2}, \ldots \ldots \ldots \ldots k_{i,n}$

**Output:** Bag-level predicted label $\widehat{Y}_i$

1: Predicting the class label and getting the attention score using Correlated MIL $f$;

2: $\widehat{Y}_i, AS_1 \leftarrow CorrMIL_1(K_i)$, where $AS_1 \in R^{1Xn}$ and $Y_i \in R^{1X1}$ ;

3: Sorting the attention score and selecting the top $n/2$ instances;

4: $\widehat{Y}_i, AS_2 \leftarrow CorrMIL_2(X_i, K_s l_i),)$, where $AS_2 \in R^{1Xn/2}$ and $Ks_i \in R^{n/2 \, X \, 512}$;

5: Sorting the attention score and selecting the top $n/4$ instances;

6: $\widehat{Y}_i \leftarrow CorrMIL_3(K_i, K_s2_i)$,, where $Ks_i \in R^{n/4 \, X \, 512}$;

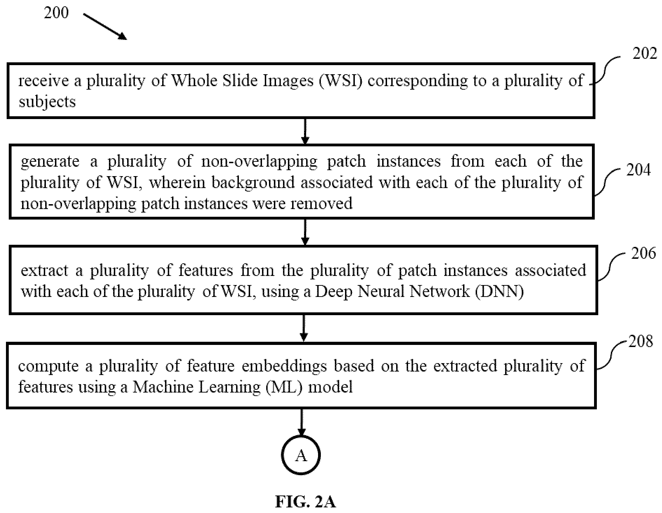

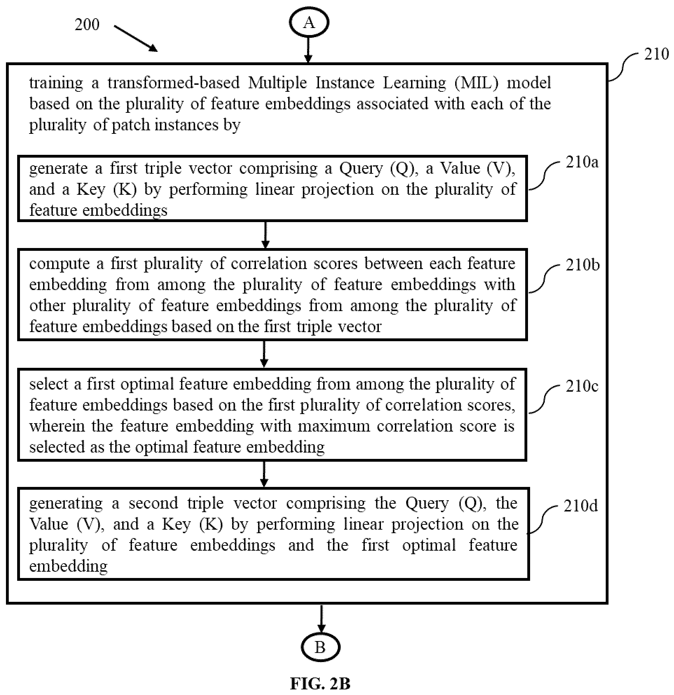

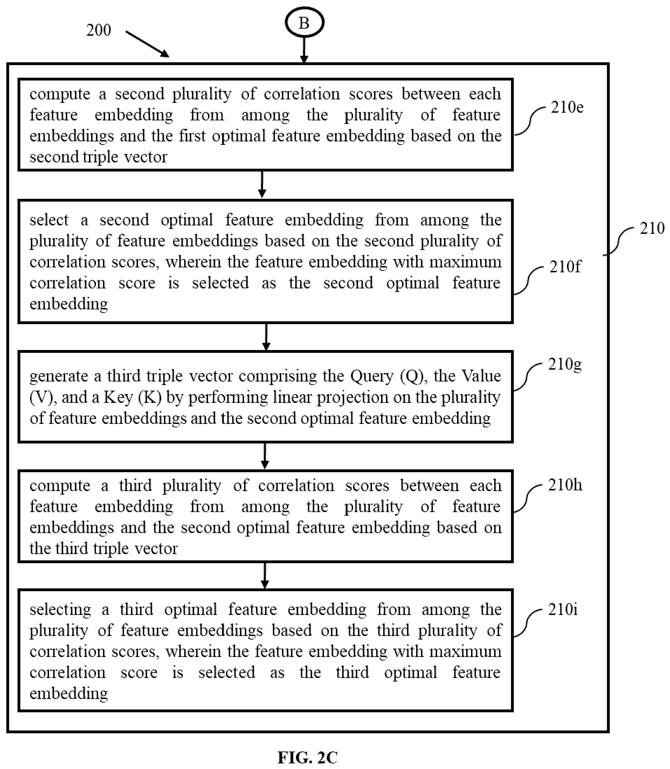

The working of the components of system 100 are explained with reference to the method steps depicted in FIGS. 2A, 2B, and 2C which is an exemplary flow diagram illustrating the efficient aggregation method 200 for multiple instance learning implemented by the system of FIG. 1A and 1B, according to some embodiments of the present disclosure. In an embodiment, the system 100 includes one or more data storage devices or the memory 104 operatively coupled to the one or more hardware processor(s) 102 and is configured to store instructions for execution of steps of the method 200 by the one or more hardware processors 102. The steps of method 200 of the present disclosure will now be explained with reference to the components or blocks of system 100 as depicted in FIG. 1A and 1B and the steps of flow diagram as depicted in FIGS. 2 A, 2B, and 2C. The method 200 may be described in the general context of computer executable instructions. Generally, computer executable instructions can include routines, programs, objects, components, data structures, procedures, modules, functions, etc., that perform particular functions or implement particular abstract data types.

The method 200 may also be practiced in a distributed computing environment where functions are performed by remote processing devices that are linked through a communication network. The order in which the method 200 is described is not intended to be construed as a limitation, and any number of the described method blocks can be combined in any order to implement the method 200, or an alternative method. Furthermore, the method 200 can be implemented in any suitable hardware, software, firmware, or combination thereof.

Now referring to FIGS. 2A, 2B, and 2C, at step 202 of method 200, the one or more hardware processors 102 are configured by the programmed instructions to receive a plurality of Whole Slide Images (WSI) corresponding to a plurality of subjects.

At step 204 of the method 200, the one or more hardware processors 102 is configured by the programmed instructions to generate a plurality of non-overlapping patch instances from each of the plurality of WSI, wherein background associated with each of the plurality of non-overlapping patch instances were removed.

At step 206 of the method 200, the one or more hardware processors 102 is configured by the programmed instructions to extract a plurality of features from the plurality of patch instances associated with each of the plurality of WSI, using a Deep Neural Network (DNN).

At step 208 of the method 200 the one or more hardware processors 102 is configured by the programmed instructions to compute a plurality of feature embeddings based on the extracted plurality of features using the DNN model.

At step 210 of the method 200, the one or more hardware processors 102 is configured by the programmed instructions to train a transformed-based Multiple Instance Learning (MIL) model based on the plurality of feature embeddings associated with each of the plurality of patch instances. The steps for training the transformed-based MIL is explained in conjunction with steps 210a through 210i.

At step 210a, (i) generating a first triple vector comprising a Query (Q), a Value (V), and a Key (K) by performing linear projection on the plurality of feature embeddings. The linear projection on the original feature embedding derives the Query (Q) and the Value (V) and the linear projection on the original feature embedding obtains the Key (K).

For example, in order to calculate $\tilde{F}$, $\tilde{B}$ and $\tilde{A}$, by using the formula depicted in pseudocode 2, its needed to obtain $\tilde{Q}$ and $\tilde{K}$, by calculating landmarks for Q and K respectively. Ultimately, multiplying the correlation matrix with the Value matrix V yields the output matrix, which represents a scaled version of the Value matrix based on the correlation scores.

At step 210b (ii) computing a first plurality of correlation scores between each feature embedding from among the plurality of feature embeddings with other plurality of feature embeddings from among the plurality of feature embeddings based on the first triple vector.

At step 210c (iii) selecting a first optimal feature embedding from among the plurality of feature embeddings based on the first plurality of correlation scores, wherein the feature embedding with maximum correlation score is selected as the optimal feature embedding.

At step 210d (iv) generating a second triple vector comprising the Query (Q), the Value (V), and a Key (K) by performing linear projection on the plurality of feature embeddings and the first optimal feature embedding. Here, the linear projection on the feature embeddings derives the Query (Q) and the Value (V) and the linear projection on the selected first optimal feature embedding obtains Key (K).

At step 210e (v) computing a second plurality of correlation scores between each feature embedding from among the plurality of feature embeddings and the first optimal feature embedding based on the second triple vector.

At step 210f (vi) selecting a second optimal feature embedding from among the plurality of feature embeddings based on the second plurality of correlation scores, wherein the feature embedding with maximum correlation score is selected as the second optimal feature embedding.

At step 210g (vii) generating a third triple vector comprising the Query (Q), the Value (V), and a Key (K) by performing linear projection on the plurality of feature embeddings and the second optimal feature embedding. Here, the linear projection on the feature embeddings derives the Query (Q) and the Value (V) and the linear projection on the second selected optimal feature embedding obtains the Key (K).

At step 210h (viii) computing a third plurality of correlation scores between each feature embedding from among the plurality of feature embeddings and the second optimal feature embedding based on the third triple vector.

At step 210i (ix) selecting a third optimal feature embedding from among the plurality of feature embeddings based on the third plurality of correlation scores, wherein the feature embedding with maximum correlation score is selected as the third optimal feature embedding.

The steps for selecting an optimal feature embedding from among the plurality of feature embeddings based on correlation scores includes the following steps (i) receiving the plurality of original feature embeddings associated with whole slide image (ii) arranging the plurality of correlation scores in the descending order for the the plurality of original feature embeddings (iii) choosing the top correlation scores among the plurality of correlation scores and (iv) selecting the first selected feature embeddings based on the chosen top correlation scores.

During inference, the one or more hardware processors 102 is configured by the programmed instructions upon receiving a WSI, predicts the bag label using the trained transformed-based MIL model explained above. The bag label prediction using the trained transformed-based MIL model helps in accurate classification of instances.

**Experimentation:** The present disclosure was experimented using three datasets to demonstrate the improved performance. For example, CAncer MEtastases in LYmph nOdes challeNge (CAMELYON)16, The Cancer Genomic, Atlas Non-small Cell Lung Cancer (TCGA-NSCLC), and The Cancer Genomic Atlas Renal Cell Carcinoma (TCGA-RCC) are the three public datasets used for conducting various experiments.

CAMELYON16 consists of a total of WSI of sentinel lymph nodes. These images were collected independently by the Radboud University Medical Center (Nijmegen, the Netherlands) and the University Medical Center Utrecht (Utrecht, the Netherlands). The training data includes 270 WSIs, with 160 being classified as normal and 110 as metastases, sourced from both universities. The test data consists of 129 WSIs collected from both institutions, with 80 being classified as normal and 49 as metastases.

TCGA-NSCLC represents the most common type of lung cancer, known as Non-Small Cell Lung Cancer (NSCLC). This dataset specifically targets two subtypes: lung adenocarcinoma (LUAD) and lung squamous cell carcinoma (LUSC). The training data comprises 840 WSIs, consisting of 433 LUAD samples and 407 LUSC samples. The test data includes 212 WSIs, with 107 LUAD samples and 105 LUSC samples.

TCGA-RCC refers to Renal Cell Carcinoma. This dataset comprises multiclass data that specifically focuses on three subclasses: Kidney Chromophobe Renal Cell Carcinoma (KICH), Kidney Renal Clear Cell Carcinoma (KIRC), and Kidney Renal Papillary Cell Carcinoma (KIRP). The training data consists of 752 WSIs, comprising 97 KICH cases, 415 KIRC cases, and 240 KIRP cases. The test data comprises 188 WSIs, including 24 KICH cases, 104 KIRC cases, and 60 KIRP cases.

Experimental Setup: In CAMELYON16, the dataset was split such that there are 270 WSIs for training and 139 image for testing. For TCGA datasets, it was first ensured that different slides from one patient do not exist in both the training and test sets, and then randomly split the data in the ratio of training:test = 80:20.

Evaluation Metrics For the evaluation metrics, accuracy and area under the curve (AUC) scores were taken into consideration to evaluate the classification performance, where the accuracy was calculated using a threshold calculated from the receiver operating characteristic curve in all experiments. For the AUC, the average AUC was used on the Camelyon16 and TCGA-NSCLC dataset, and the average one versus-rest AUC (macro-averaged) was used on the TCGA-RCC dataset. All the results over TCGA datasets and Camelyon16 are obtained by 4-fold cross validation

Implementation Details: Each WSI has images stored at different magnification levels like 5x, 10x and 20x. For our analysis, 10x magnification was chosen as 5x would lose the fine granular clarity of cells and 20x might lose the over all pattern of tumor. So using OpenSlide, each WSI is converted in non-overlapping patches, out of which background patches where removed. This patches where further converted into embedding of 512 dimension using ResNet-18 with pre-trained weights on 228 ImageNet dataset. As a result, each bag can be represented as $\hat{X}_i \in R\ n \times 512$. In the training step, cross-entropy loss was adopted, and the Adam optimizer was employed with a learning rate of 2e-4 and weight decay of 1e-5. The size of mini-batch (B) is 1. While calculating the evaluation matrix, softmax is used to 232 233 normalize the predicted scores for each class.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of the present disclosure herein address the unresolved problem of selecting positive instances and correlate the features of those selected instances with other instances. As a result, with the present disclosure, it was observed that the differentiation of the negative instances from the selected positive instances helps in classifying those uncertain instances, thus classifying into either of the classes and lower down the importance of negative instances. Further, the present disclosure focuses on enhancing the sub-classification of tumors rather than distinguishing between normal tissue and cancerous tumor tissue.

It is to be understood that the scope of the protection is extended to such a program and in addition to a computer-readable means having a message therein such computer-readable storage means contain program-code means for implementation of one or more steps of the method when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g. an ASIC and an FPGA, or at least one microprocessor and at least one memory with software modules located therein. Thus, the means can include both hardware means, and software means. The method embodiments described herein could be implemented in hardware and software. The device may also include software means. Alternatively, the embodiments may be implemented on different hardware devices, e.g., using a plurality of CPUs, GPUs and edge computing devices.

The embodiments herein can comprise hardware and software elements. The embodiments that are implemented in software include but are not limited to, firmware, resident software, microcode, etc. The functions performed by various modules described herein may be implemented in other modules or combinations of other modules. For the purposes of this description, a computer-usable or computer readable medium can be any apparatus that can comprise, store, communicate, propagate, or transport the program for use by or in connection with the instruction execution system, apparatus, or device. The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise. Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e. non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor-implemented method (200), the method comprising:

receiving (202), by one or more hardware processors, a plurality of Whole Slide Images (WSIs) corresponding to a plurality of subjects;

generating (204), by the one or more hardware processors, a plurality of non-overlapping patch instances from each of the plurality of WSIs, wherein a background associated with each of the plurality of non-overlapping patch instances were removed;

extracting (206), by the one or more hardware processors, a plurality of features from the plurality of patch instances associated with each of the plurality of WSI, using a Deep Neural Network (DNN);

computing (208), by the one or more hardware processors, a plurality of feature embeddings based on the extracted plurality of features using a Machine Learning (ML) model; and

training (210), by the one or more hardware processors, a transformed-based Multiple Instance Learning (MIL) model based on the plurality of feature embeddings associated with each of the plurality of patch instances by:

generating (210a) a first triple vector comprising a Query (Q), a Value (V), and a Key (K) by performing linear projection on the plurality of feature embeddings;

computing (210b) a first plurality of correlation scores between each feature embedding from among the plurality of feature embeddings with other plurality of feature embeddings from among the plurality of feature embeddings based on the first triple vector;

selecting (210c) a first optimal feature embedding from among the plurality of feature embeddings based on the first plurality of correlation scores, wherein the feature embedding with maximum correlation score is selected as the optimal feature embedding;

generating (210d) a second triple vector comprising the Query (Q), the Value (V), and a Key (K) by performing linear projection on the plurality of feature embeddings and the first optimal feature embedding;

computing (210e) a second plurality of correlation scores between each feature embedding from among the plurality of feature embeddings and the first optimal feature embedding based on the second triple vector;

selecting (210f) a second optimal feature embedding from among the plurality of feature embeddings based on the second plurality of correlation scores, wherein the feature embedding with maximum correlation score is selected as the second optimal feature embedding;

generating (210g) a third triple vector comprising the Query (Q), the Value (V), and a Key (K) by performing linear projection on the plurality of feature embeddings and the second optimal feature embedding;

computing (210h) a third plurality of correlation scores between each feature embedding from among the plurality of feature embeddings and the second optimal feature embedding based on the third triple vector; and

selecting (210i) a third optimal feature embedding from among the plurality of feature embeddings based on the third plurality of correlation scores, wherein the feature embedding with maximum correlation score is selected as the third optimal feature embedding.

2. The processor implemented method as claimed in claim 1, during inference, the trained transformed-based MIL model predicts the bag label on receiving the WSI.

3. The processor implemented method as claimed in claim 1, wherein steps for selecting an optimal feature embedding from among the plurality of feature embeddings based on correlation scores comprises:

receiving the plurality of original feature embeddings associated with the WSI;

arranging the plurality of correlation scores in the descending order for the plurality of original feature embeddings;

choosing the top correlation scores among the plurality of correlation scores; and

selecting the first selected feature embeddings based on the chosen top correlation scores.

4. A system (100) comprising:

at least one memory (104) storing programmed instructions; one or more Input /Output (I/O) interfaces (112); and one or more hardware processors (102) operatively coupled to the at least one memory (104), wherein the one or more hardware processors (102) are configured by the programmed instructions to:

receive a plurality of Whole Slide Images (WSIs) corresponding to a plurality of subjects;

generate a plurality of non-overlapping patch instances from each of the plurality of WSIs, wherein a background associated with each of the plurality of non-overlapping patch instances were removed;

extract a plurality of features from the plurality of patch instances associated with each of the plurality of WSI, using a Deep Neural Network (DNN);

compute a plurality of feature embeddings based on the extracted plurality of features using a Machine Learning (ML) model; and
train a transformed-based Multiple Instance Learning (MIL) model based on the plurality of feature embeddings associated with each of the plurality of patch instances by:

generating a first triple vector comprising a Query (Q), a Value (V), and a Key (K) by performing linear projection on the plurality of feature embeddings;
computing a first plurality of correlation scores between each feature embedding from among the plurality of feature embeddings with other plurality of feature embeddings from among the plurality of feature embeddings based on the first triple vector;
selecting a first optimal feature embedding from among the plurality of feature embeddings based on the first plurality of correlation scores, wherein the feature embedding with maximum correlation score is selected as the optimal feature embedding;
generating a second triple vector comprising the Query (Q), the Value (V), and a Key (K) by performing linear projection on the plurality of feature embeddings and the first optimal feature embedding;
computing a second plurality of correlation scores between each feature embedding from among the plurality of feature embeddings and the first optimal feature embedding based on the second triple vector;
selecting a second optimal feature embedding from among the plurality of feature embeddings based on the second plurality of correlation scores, wherein the feature embedding with maximum correlation score is selected as the second optimal feature embedding;
generating a third triple vector comprising the Query (Q), the Value (V), and a Key (K) by performing linear projection on the plurality of feature embeddings and the second optimal feature embedding;
computing a third plurality of correlation scores between each feature embedding from among the plurality of feature embeddings and the second optimal feature embedding based on the third triple vector; and
selecting a third optimal feature embedding from among the plurality of feature embeddings based on the third plurality of correlation scores, wherein the feature embedding with maximum correlation score is selected as the third optimal feature embedding.

5. The system of claim 4, during inference, the trained transformed-based MIL model predicts the bag label on receiving the WSI.

6. The system of claim 4, wherein steps for selecting an optimal feature embedding from among the plurality of feature embeddings based on correlation scores comprises:

receiving the plurality of original feature embeddings associated with the WSI;
arranging the plurality of correlation scores in the descending order for the plurality of original feature embeddings;
choosing the top correlation scores among the plurality of correlation scores; and
selecting the first selected feature embeddings based on the chosen top correlation scores.

7. One or more non-transitory machine-readable information storage mediums comprising one or more instructions which when executed by one or more hardware processors cause:

receiving, a plurality of Whole Slide Images (WSIs) corresponding to a plurality of subjects;
generating, a plurality of non-overlapping patch instances from each of the plurality of WSIs, wherein a background associated with each of the plurality of non-overlapping patch instances were removed;
extracting, a plurality of features from the plurality of patch instances associated with each of the plurality of WSI, using a Deep Neural Network (DNN);
computing, a plurality of feature embeddings based on the extracted plurality of features using a Machine Learning (ML) model; and
training, a transformed-based Multiple Instance Learning (MIL) model based on the plurality of feature embeddings associated with each of the plurality of patch instances by:

generating a first triple vector comprising a Query (Q), a Value (V), and a Key (K) by performing linear projection on the plurality of feature embeddings;
computing a first plurality of correlation scores between each feature embedding from among the plurality of feature embeddings with other plurality of feature embeddings from among the plurality of feature embeddings based on the first triple vector;

selecting a first optimal feature embedding from among the plurality of feature embeddings based on the first plurality of correlation scores, wherein the feature embedding with maximum correlation score is selected as the optimal feature embedding;

generating a second triple vector comprising the Query (Q), the Value (V), and a Key (K) by performing linear projection on the plurality of feature embeddings and the first optimal feature embedding;

computing a second plurality of correlation scores between each feature embedding from among the plurality of feature embeddings and the first optimal feature embedding based on the second triple vector;

selecting a second optimal feature embedding from among the plurality of feature embeddings based on the second plurality of correlation scores, wherein the feature embedding with maximum correlation score is selected as the second optimal feature embedding;

generating a third triple vector comprising the Query (Q), the Value (V), and a Key (K) by performing linear projection on the plurality of feature embeddings and the second optimal feature embedding;

computing a third plurality of correlation scores between each feature embedding from among the plurality of feature embeddings and the second optimal feature embedding based on the third triple vector; and

selecting a third optimal feature embedding from among the plurality of feature embeddings based on the third plurality of correlation scores, wherein the feature embedding with maximum correlation score is selected as the third optimal feature embedding.

8. The one or more non-transitory machine readable information storage mediums as claimed in claim 7, during inference, the trained transformed-based MIL model predicts the bag label on receiving the WSI.

9. The one or more non-transitory machine readable information storage mediums as claimed in claim 7, wherein steps for selecting an optimal feature embedding from among the plurality of feature embeddings based on correlation scores comprises:

receiving the plurality of original feature embeddings associated with the WSI;

arranging the plurality of correlation scores in the descending order for the plurality of original feature embeddings;

choosing the top correlation scores among the plurality of correlation scores; and

selecting the first selected feature embeddings based on the chosen top correlation scores.

100

MEMORY 104

MODULES 106

REPOSITORY 110

108

I/O INTERFACE
112

HARDWARE
PROCESSORS
102

FIG. 1A

512

ResNet-18

N

Whole Slide Image

Extracted patches

Feature Extraction

Feature Embeddings

A

FIG. 1B

FIG. 1C

**Transformer based
Correaltion MIL Module**

FIG. 1D

**Selection module**

FIG. 1E

FIG. 1F

200

receive a plurality of Whole Slide Images (WSI) corresponding to a plurality of subjects ⌒ 202

generate a plurality of non-overlapping patch instances from each of the plurality of WSI, wherein background associated with each of the plurality of non-overlapping patch instances were removed ⌒ 204

extract a plurality of features from the plurality of patch instances associated with each of the plurality of WSI, using a Deep Neural Network (DNN) ⌒ 206

compute a plurality of feature embeddings based on the extracted plurality of features using a Machine Learning (ML) model ⌒ 208

( A )

**FIG. 2A**

200

(A)

⌐ 210

training a transformed-based Multiple Instance Learning (MIL) model based on the plurality of feature embeddings associated with each of the plurality of patch instances by

generate a first triple vector comprising a Query (Q), a Value (V), and a Key (K) by performing linear projection on the plurality of feature embeddings

⌐ 210a

compute a first plurality of correlation scores between each feature embedding from among the plurality of feature embeddings with other plurality of feature embeddings from among the plurality of feature embeddings based on the first triple vector

⌐ 210b

select a first optimal feature embedding from among the plurality of feature embeddings based on the first plurality of correlation scores, wherein the feature embedding with maximum correlation score is selected as the optimal feature embedding

⌐ 210c

generating a second triple vector comprising the Query (Q), the Value (V), and a Key (K) by performing linear projection on the plurality of feature embeddings and the first optimal feature embedding

⌐ 210d

(B)

**FIG. 2B**

200

(B)

compute a second plurality of correlation scores between each feature embedding from among the plurality of feature embeddings and the first optimal feature embedding based on the second triple vector

210e

select a second optimal feature embedding from among the plurality of feature embeddings based on the second plurality of correlation scores, wherein the feature embedding with maximum correlation score is selected as the second optimal feature embedding

210f

210

generate a third triple vector comprising the Query (Q), the Value (V), and a Key (K) by performing linear projection on the plurality of feature embeddings and the second optimal feature embedding

210g

compute a third plurality of correlation scores between each feature embedding from among the plurality of feature embeddings and the second optimal feature embedding based on the third triple vector

210h

selecting a third optimal feature embedding from among the plurality of feature embeddings based on the third plurality of correlation scores, wherein the feature embedding with maximum correlation score is selected as the third optimal feature embedding

210i

**FIG. 2C**

# EUROPEAN SEARCH REPORT

**Application Number**

EP 25 20 2781

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHUCHEN SHAO ET AL: "TransMIL: Transformer based Correlated Multiple Instance Learning for Whole Slide Image Classification", ARXIV.ORG, CORNELL UNIVERSITY LIBRARY, 201 OLIN LIBRARY CORNELL UNIVERSITY ITHACA, NY 14853, 31 October 2021 (2021-10-31), XP091069066, * Algorithms 1-3, Sections 3.2, 3.3, and 4, Appendix B; figures 1, 3 * | 1-9 | INV. G06F18/28 G06V10/82 G06V20/69 |
| X,P | MAKADIYA SHWET ET AL: "SS-MIL: Attention-Based Selective Correlated Multiple Instance Learning for Whole Slide Image Classification", 12 May 2025 (2025-05-12), COMPUTER VISION - ECCV 2024 WORKSHOPS; [LECTURE NOTES IN COMPUTER SCIENCE; LECT.NOTES COMPUTER], SPRINGER NATURE SWITZERLAND, CHAM, PAGE(S) 239 - 251, XP047727694, ISSN: 0302-9743 ISBN: 978-3-031-91720-2 [retrieved on 2025-05-12] * the whole document * | 1-9 | |

**TECHNICAL FIELDS SEARCHED (IPC)**

G06F
G06V

The present search report has been drawn up for all claims

| Place of search | Date of completion of the search | Examiner |
|---|---|---|
| The Hague | 14 January 2026 | Grigorescu, Simona |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421073366